# EUROPEAN PATENT APPLICATION

(11) **EP 4 014 956 A1**
(43) Date of publication of application: **22.06.2022**
(21) Application number: 20215216.1
(22) Date of filing: 17.12.2020
(51) Int. Cl.: A61K 8/34, A61K 8/37, A61K 8/73, A61Q 19/00, A61Q 17/04, A61Q 1/02

(54) **TRANSPARENT GEL**

(71) Applicant: Oleon N.V., 9940 Ertvelde (BE)
(72) Inventor: PEETERS, Hilde, 3140 KEERBERGEN (BE)
(74) Representative: Santarelli

(57) **Abstract**

The present invention relates to a combination comprising alcohols, glyceryl mono fatty acid ester(s) and ethyl cellulose polymer(s), its use as thickener and/or gelling agent and to the resulting cosmetic products.

## Description

The present invention relates to a specific combination, its use as a thickener, gelling agent and/or shear-thinning agent, and compositions comprising it. This specific combination presents the advantage of being transparent and this transparency lasts even after mixing with other compounds, which is particularly wanted in the field of cosmetics.

Indeed, the transparent appearance of cosmetic products has a commercial interest, since for customers it is a guarantee of cleanliness and pureness. It is also easier for the formulator to control the homogeneity of the cosmetic product.

Current transparent thickeners, gelling agents and/or shear-thinning agents used in cosmetics are based on a polymer or a molecule that generally needs to be heated to a high temperature (often higher than 110°C) for melting down or becoming pourable.

But, to prepare a cosmetic product, the formulator is limited to temperatures below 100°C because of the use of thermal sensitive compounds such as preservatives and some antioxidants, and because of the water bath usually used for heating in this field.

Therefore, there is still a need for a thickener, a gelling agent and/or a shear-thinning agent that would present one or more, and preferably all of the following characteristics:
- transparent;
- stable;
- pourable at a temperature of 90°C, i.e. having a dynamic viscosity at a shear rate of 10 s⁻¹ of less than 15000 mPa.s at a temperature of 90°C;
- spreadable;
- compatible with different types of cosmetics compounds;
thus allowing cosmetic formulators to prepare cosmetic products at a temperature of at most 90°C.

The inventors surprisingly found that a specific combination may present all of those characteristics with the additional advantage of being in the form of gel at a temperature up to 50°C or even higher temperatures, allowing thus the preparation of additional possible cosmetics products such as lipsticks which need to resist to temperatures up to 50°C.

Accordingly, the present invention relates to a combination comprising or consisting of:
- a dimer diol;
- branched primary alcohol(s) of Formula (I), 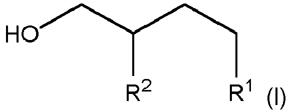 wherein R¹ and R², identical or different, are linear or branched alkyl groups, comprising from 3 to 16 carbon atoms;
- at least 5% by weight of a glyceryl mono fatty acid ester; and
- at least 3% by weight of an ethyl cellulose polymer;
wherein the dimer diol and the branched primary alcohol(s) of Formula (I) are liquid at 25°C and atmospheric pressure; and
wherein the total quantity of dimer diol and branched primary alcohol of Formula (I) is of at least 70% by weight;
weight percentages being based on the weight of the combination.

A dimer diol is a mixture of cyclic and noncyclic isomers, known by the skilled person. It is usually obtained by:
- dimerization of unsaturated fatty acid(s) or ester(s) thereof followed by reduction of respectively acid or ester groups into hydroxyl groups, or
- dimerization of unsaturated fatty alcohol(s).

Advantageously, unsaturated fatty acid(s) or ester(s) thereof and unsaturated fatty alcohol(s) are respectively mono carboxylic acid(s) or ester(s) and mono alcohol(s), each hydrocarbon chain of fatty acid(s) or fatty alcohol(s) comprising between 18 and 24, preferably between 18 and 22 carbon atoms. Also, dimer diol molecules preferably comprise between 36 and 48, more preferably between 36 and 44 carbon atoms.

In the present application, unless otherwise indicated, all ranges of values used are to be understood as being inclusive limits.

Preferably, the dimer diol comprises more than 50% by weight, more preferably more than 70% by weight of noncyclic isomers, percentages by weight being given on the total weight of the dimer diol. By total weight of the dimer diol, it is intended the weight of all dimer diol molecules.

Advantageously, the dimer diol comprises 36 carbon atoms. In particular, the dimer diol is prepared from unsaturated fatty acid(s) or unsaturated fatty alcohol(s) comprising 18 carbon atoms.

In the branched primary alcohol(s) of Formula (I), the alkyl groups are preferably groups consisting of carbon and hydrogen atoms.

Preferably, the alkyl groups R1 and R2 are saturated.

The alkyl groups may be a propyl, butyl, pentyl, hexyl, heptyl, octyl, nonyl, decyl, undecyl, dodecyl, tridecyl, tetradecyl, pentadecyl or octadecyl group or any of their isomers.

Advantageously, a branched primary alcohol of Formula (I) is obtainable by the condensation of two primary alcohol monomers according to a Guerbet reaction.

The Guerbet reaction is well known in the art. The Guerbet reaction comprises the condensation of two, identical or different, primary alcohol monomers with elimination of water. It typically proceeds in the presence of a base such as NaOH or KOH and/or a catalyst such as palladium, nickel, copper or zinc, at a temperature of at least 120°C, to form a primary alcohol dimer which is a beta-alkylated primary alcohol.

The primary alcohol monomer may be linear or branched.

Preferably the primary alcohol monomer is not substituted by any heteroatom other than the oxygen of the hydroxyl group.

Preferably, the primary alcohol monomer is saturated.

In case of two identical primary alcohol monomers are used, then a branched primary alcohol of Formula (I) with identical R¹ and R² is formed.

In case of at least two different primary alcohol monomers are used, then a mixture of branched primary alcohols of Formula (I) may be formed, with a least one branched primary alcohol of Formula (I) with different R¹ and R².

Preferably, branched primary alcohol(s) of Formula (I) is/are obtainable from primary alcohol monomer(s) chosen from the group consisting of pentanol, 3-methylbutanol, 2-methylbutanol, hexanol, 3-methylpentanol, 4-methylpentanol, heptanol, 2-ethylhexanol, octanol, nonanol, isononanol, decanol, isodecanol, undecanol, dodecanol, tridecanol, tetradecanol, pentadecanol, hexadecanol, heptadecanol, octadecanol, isooctadecanol and their mixtures.

Preferably, branched primary alcohol(s) of Formula (I) is selected from the group consisting of 2-isopropyl-5-methylhexanol, 2-octyl-dodecanol, 2-octyl-decanol, 2-hexyl-dodecanol, 2-hexyl-decanol, isohexatriacontanol and their mixtures.

Such a combination according to the invention is odorless and colorless and thus particularly adapted for applications in cosmetics. Moreover, the combination presents particularly good skin feel.

A glyceryl mono fatty acid ester is also named monoglyceride of fatty acid.

The fatty acid of the glyceryl mono fatty acid ester, may be saturated or unsaturated and preferably comprises from 4 to 24, more preferably from 4 to 18 carbon atoms.

The combination according to the invention may comprise more than one glyceryl mono fatty acid ester, such as a mixture of glyceryl mono fatty acid esters, wherein each fatty acid independently comprises from 4 to 24, more preferably from 4 to 18 carbon atoms.

Preferably the fatty acid is straight.

An ethyl cellulose polymer is a derivative of cellulose, wherein some hydroxyl groups on the repeating glucose units are converted into ethoxyl groups. In particular, the ethyl cellulose polymer is of formula (I): 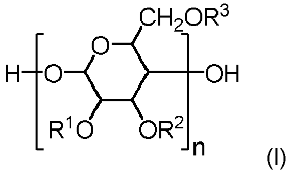 wherein
- R¹, R² and R³, identical or different, represent H or CH₂CH₃, and
- n, an integer, is of at least 2.

In commercially available ethyl cellulose polymers, ethoxyl content ranges from 45 to 51%, preferably from 48 to 49.5% by weight, based on the weight of ethyl cellulose polymer. Ethyl cellulose polymers are characterized by their viscosity at 25°C when they are diluted at a concentration of 5% by weight on the weight of a solution comprising a 80:20 mixture of toluene:ethanol (wt/wt). Ethyl cellulose polymers having a viscosity comprised between 4 mPa.s and 350 mPa.s can be used in the present invention, preferably, ethyl cellulose polymers having a viscosity comprised between 20 mPa.s and 300 mPa.s, more preferably, ethyl cellulose polymers having a viscosity comprised between 45 mPa.s and 200 mPa.s, even more preferably around 100 mPa.s.

Alternatively, a mixture of ethyl cellulose polymers having different viscosity can be used.

Preferably, the total quantity of dimer diol and branched primary alcohol(s) of Formula (I) is of at least 75% by weight, more preferably at least 80% by weight based on the weight of the combination.

By "total quantity of dimer diol and branched primary alcohol(s) of Formula (I)", it is intended the weight of all dimer diol and branched primary alcohol(s) of Formula (I) molecules.

Preferably, the dimer diol and the branched primary alcohol(s) of Formula (I) are liquid at 23°C, more preferably at 20°C, and atmospheric pressure.

Advantageously, in the combination according to the invention, the weight ratio total quantity of branched primary alcohol(s) of Formula (I) / quantity of dimer diol is comprised between 0.5 and 2.2.

Preferably, the quantity of dimer diol is of at least 25 wt%, more preferably at least 28 wt%, even more preferably at least 30 wt% based on the weight of the combination.

Preferably, the quantity of dimer diol is of at most 50 wt%, more preferably at most 40 wt% based on the weight of the combination.

Preferably, the total quantity of branched primary alcohol(s) of Formula (I) is of at least 25 wt%, more preferably at least 35 wt%, even more preferably at least 40 wt% based on the weight of the combination.

Preferably, the total quantity of branched primary alcohol(s) of Formula (I) is of at most 60 wt%, more preferably at most 55 wt% based on the weight of the combination.

By "total quantity of branched primary alcohol(s) of Formula (I)", it is intended the weight of all branched primary alcohol(s) of Formula (I) molecules.

Preferably, the total quantity of glyceryl mono fatty acid ester(s) is of at most 15 wt%, more preferably at most 12 wt% based on the weight of the combination.

Preferably, the total quantity of glyceryl mono fatty acid ester(s) is comprised between 5 and 15 wt%, more preferably between 5 and 12 wt% based on the weight of the combination.

By "total quantity of glyceryl mono fatty acid ester(s)", it is intended the weight of all glyceryl mono fatty acid ester(s) molecules.

Preferably, the total quantity of ethyl cellulose polymer(s) is of at least 4 wt%, more preferably at least 5 wt%, even more preferably at least 6 wt% based on the weight of the combination.

Preferably, the total quantity of ethyl cellulose polymer(s) is of at most 15 wt%, more preferably at most 10 wt%, even more preferably at most 8 wt% based on the weight of the combination.

By "total quantity of ethyl cellulose polymer(s)", it is intended the quantity of all ethyl cellulose polymer(s) molecules.

The total quantity of ethyl cellulose polymer(s) can be adapted to the viscosity desired.

Preferably, the combination according to the invention comprises between 4 and 15 wt%, more preferably between 5 and 15 wt%, even more preferably between 6 and 12 wt% of an ethyl cellulose polymer having a viscosity comprised between 45 mPa.s and 200 mPa.s.

More particularly, the combination according to the invention comprises between 5 and 10 wt%, preferably between 6 and 8 wt% of an ethyl cellulose polymer having a viscosity of 100 mPa.s.

Preferably, the weight ratio total quantity of branched primary alcohol(s) of Formula (I) / quantity of dimer diol is comprised between 1 and 2.

Preferably, the total quantity of branched primary alcohol(s) of Formula (I) is higher than the quantity of dimer diol. Thus, the combinations according to the invention exhibit better skin feel.

Thus, the weight ratio total quantity of branched primary alcohol(s) of Formula (I) / quantity of dimer diol is preferably comprised between 1.2 and 1.8, more preferably between 1.3 and 1.7, even more preferably between 1.4 and 1.6.

At 25°C +/-5°C, a combination according to the invention is liquid, as long as the combination is not heated at a temperature higher than the glass transition temperature of the ethyl cellulose polymer present in the combination. If a mixture of ethyl cellulose polymers is used, then the combination remains liquid if it is not heated at a temperature higher than the highest glass transition temperature of ethyl cellulose polymers.

Thus, the present invention also relates to a combination according to the invention, which is in the form of a gel.

The combination according to the invention in the form of a transparent gel is stable over time, such as during at least 3 months at 20°C, preferably during at least 3 months at 60°C, as demonstrated in Example 2.3.1.

By stable, it is intended that the appearance remains a gel without any separation such as formation of a liquid layer of any component of the combination on the top of the gel.

Moreover, the combination in the form of a gel is stable to temperature changes. The combination can be heated until the gel melts, thus becoming liquid, and when cooled down, the combination recovers its initial viscosity and appearance. Its appearance remains a gel at room temperature after repetition of steps of heating and cooling as illustrated in Example 2.3.2.

Depending on the viscosity of the ethyl cellulose polymer(s) and its quantity in the combination, depending on the quantity of dimer diol and depending on the glyceryl mono fatty acid ester(s) used, combinations according to the invention in the form of a gel with a large range of viscosity can be obtained. Dynamic viscosity of combinations in the form of a gel at 25°C and at a shear rate of 10 s⁻¹, may be more than 100 Pa.s. However, as the viscosity of combinations is going down when the temperature increases, it is possible to heat the more viscous combinations, preferably at a temperature of about 80°C, to render them pourable, as shown in Example 2.2.

A preferred combination in the form of a gel comprises or consists of:
- 25-40 wt% of a dimer diol;
- 40-60 wt% of branched primary alcohol(s) of Formula (I);
- 5-15 wt% of glyceryl mono fatty acid ester(s);
- 3-15 wt% of ethyl cellulose polymer(s),
wherein the dimer diol and the branched primary alcohol(s) of Formula (I) are liquid at 25°C and atmospheric pressure; and
wherein the total quantity of dimer diol and branched primary alcohol(s) of Formula (I) is of at least 70% by weight;
wherein the weight ratio total quantity of branched primary alcohol(s) of Formula (I) / quantity of dimer diol is comprised between 1.2 and 1.8, preferably between 1.3 and 1.7, even more preferably between 1.4 and 1.6;
weight percentages being based on the total weight of the combination.

In a particularly preferred combination, the dimer diol comprises 36 carbon atoms. Preferably, the ethyl cellulose polymer used in the particularly preferred combination according to the invention has a viscosity of 100 mPa.s (as defined above).

The combinations according to the invention present some rheological properties. In particular, the combinations present shear-thinning and thixotropic properties.

By shear-thinning, it is intended a compound or a mixture of compounds whose viscosity decreases when it is subjected to an increasing stress, such as a shear rate. The shear-thinning property of the different combinations is shown in Example 2.4, on Table 4.

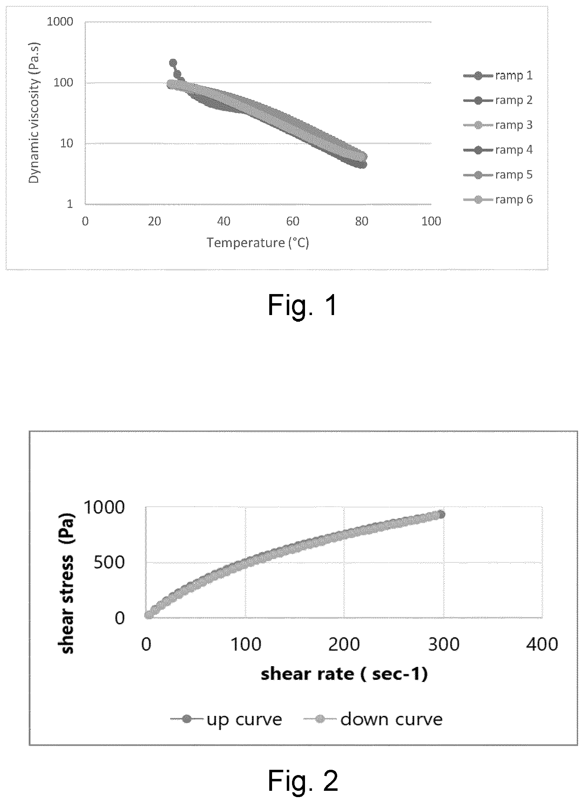

By thixotropic, it is intended a compound or a mixture of compounds whose viscosity decreases when it is subjected to an increasing shear rate, and whose viscosity is recovered when shear ceases. As shown in Figure 2, when the shear rate applied to a combination according to the invention is reduced, the stress path returns to the initial shear stress value, which is characteristic of most thixotropic materials.

Combinations according to the invention recover their initial viscosity and their gel texture after shear ceases.

The present application also relates to a process for preparing a combination according to the invention, which comprises a step of mixing the dimer diol, the branched primary alcohol(s), the glyceryl mono fatty acid ester(s), and an ethyl cellulose polymer.

The present application also relates to a process for preparing a combination according to the invention in the form of a gel, which comprises a step of heating the combination at a temperature higher than the glass transition temperature of the ethyl cellulose polymer, followed by a step of cooling the combination to form a transparent gel.

The dimer diol, the branched primary alcohol(s) of Formula (I), the glyceryl mono fatty acid ester(s) and the ethyl cellulose polymer are as described above, including preferential and advantageous features.

In a first embodiment, the process for preparing a combination according to the invention in the form of a gel comprises the following steps:
i) mixing a dimer diol, branched primary alcohol(s), glyceryl mono fatty acid ester(s), and an ethyl cellulose polymer to obtain a combination;
ii) heating the combination under stirring at a temperature higher than the glass transition temperature of the ethyl cellulose polymer; and
iii) cooling down the combination to obtain a combination in the form of a gel.

In a second advantageous embodiment, the dimer diol, the branched primary alcohol(s) and the glyceryl mono fatty acid ester(s) are heated until homogeneous, prior to addition of ethyl cellulose polymer.

According to this second embodiment, gels present higher viscosities than those obtained according to the first embodiment.

Thus, the invention concerns a process for preparing a combination in the form of a gel according to the invention comprising the following steps:
i) mixing the dimer diol, branched primary alcohol(s) and glyceryl mono fatty acid ester(s) at a temperature of at least 40°C;
ii) adding the ethyl cellulose polymer to obtain a combination;
iii) heating the combination under stirring at a temperature higher than the glass transition temperature of the ethyl cellulose polymer; and
iv) cooling down the combination to obtain a combination in the form of a gel.

In step i), the temperature is adapted according to the melting point of the glyceryl mono fatty acid ester(s) used, which should be higher than the melting point of the glyceryl mono fatty acid ester, or if a mixture of glyceryl mono fatty acid esters is used, higher than the highest melting point of the glyceryl mono fatty acid esters.

Step i) is preferably conducted until all the components are totally solubilized and homogeneously mixed.

Addition of ethyl cellulose polymer in step ii) is preferably not done in one time. The ethyl cellulose polymer is preferably added over a given time, by continuous addition over time or by discontinuous addition, i.e. at least 2 additions separated by a period of time.

If more than one ethyl cellulose polymer is used in the combination, then step iii) is conducted at a temperature higher than the highest glass transition temperature of the ethyl cellulose polymers.

Step iii) is preferably conducted until all the ethyl cellulose polymer(s) is totally solubilized.

In step iv), the combination is preferably cooled down at a rate of 0.6°C/min or lower. This slow cooling down is controlled until reaching a temperature of 60°C, preferably until 50°C, more preferably until 45°C.

Step iv) is preferably conducted without stirring. Alternatively, the stirring is decreased once the viscosity becomes higher than 25000 mPa.s, to avoid incorporation of air bubbles.

The combination can be shacked during the cooling in order to release the air bubbles.

Besides the advantage of being a transparent, the combinations according to the invention in the form of a gel present shear-thinning and thixotropic properties.

Thus, the combinations of the invention find advantageously applications in cosmetics, such as thickener, gelling agent and/or a shear-thinning agent.

The present invention also concerns the use of a combination according the invention, as a thickener.

The combination may thicken a liquid compound, in particular, a liquid organic compound and/or a liquid silicone.

Thus the present invention also concerns a process for increasing the viscosity of a liquid organic compound and/or a liquid silicone, by mixing a combination according to the invention, with said liquid organic compound and/or liquid silicone.

The liquid organic compound and/or the liquid silicone is/are liquid at 25°C and atmospheric pressure.

The liquid organic compound comprises carbon and hydrogen atoms, and optionally an acid, an alcohol, an ester, an ether, a ketone and/or a nitrile group.

Thus, the liquid organic compound may be a mineral oil, a terpene, a fatty acid, an alcohol, an ester or a compound comprising at least two different functional groups.

Preferably, the liquid organic compound and the liquid silicone are chosen among compounds used in the field of cosmetics.

In particular, the liquid silicone may be an emollient, and the liquid organic compound may be a humectant, an emulsifier, an emollient, an organic UV filter, an insect repellent, a perfume or mixtures thereof.

More particularly, the humectant is selected from the group consisting of propylene glycol, glycerol and 1,3 butylene glycol.

More particularly, the emulsifier is selected from the group consisting of polysorbate 20, polysorbate 80, polyglyceryl-3 diisostearate, mono and di-glyceride C18 unsaturated citrates and sorbitan mono oleate.

More particularly, the emollient is selected from the group consisting of triolein, isoamyl laurate, propylene glycol dicaprylate/dicaprate, medium chain triglycerides (MCT), paraffin highly liquid, octyldodecanol, dimethicones, cyclopentasiloxane and caprylyl methicone.

More particularly, the organic UV filter is selected from the group consisting of octocrylene, octyl methoxycinnamate, homosalate and ethylhexyl salicylate.

More particularly, the insect repellent is selected from the group consisting of ethyl butylacetylaminopropionate, citronellol, geraniol and citronella.

Consequently, the liquid organic compound is preferably selected from the group consisting of propylene glycol, glycerol, 1,3 butylene glycol, polysorbate 20, polysorbate 80, polyglyceryl-3 diisostearate, mono and di-glyceride C18 unsaturated citrates, sorbitan mono oleate, triolein, isoamyl laurate, propylene glycol dicaprylate/dicaprate, medium chain triglycerides (MCT), paraffin highly liquid, octyldodecanol, caprylyl methicone, octocrylene, octyl methoxycinnamate, homosalate, ethylhexyl salicylate, ethyl butylacetylaminopropionate, citronellol, geraniol, citronella and mixtures thereof.

Consequently, the liquid silicone is preferably selected from the group consisting of dimethicones, cyclopentasiloxane and caprylyl methicone.

The present invention also concerns the use of a combination according to the invention, as a shear-thinning agent.

When a combination according to the invention is mixed with a liquid organic compound and/or a liquid silicone, the resulting gel composition presents shear-thinning property.

Thus the present invention also concerns a process for decreasing the viscosity under shear-stress of a liquid organic compound and/or a liquid silicone, by mixing a combination according to the invention with said liquid organic compound and/or liquid silicone.

Advantageously, the combinations according to the invention exhibit both properties, it can be used as a thickener and as a shear-thinning agent. Thus, the combinations according to the invention can be used as a rheology modifier.

The present invention also concerns the use of a combination according to the invention, as a gelling agent.

The present invention also concerns a process for gelling a liquid organic compound and/or a liquid silicone, comprising a step of mixing and heating a combination according to any of claims 1 to 3, with said liquid organic compound and/or liquid silicone.

The gel obtained is transparent.

In a fist embodiment of the above processes (increasing the viscosity, decreasing the viscosity under shear-stress, gelling), the combination according to the invention is not in the form of a gel. The combination is first mixed with the liquid organic compound and/or the liquid silicone to obtain a composition, the latter being then further heated at a temperature higher than the glass transition temperature of the ethyl cellulose polymer present in the combination, or if more than one ethyl cellulose polymer is used, at a temperature higher than the highest glass transition temperature of the ethyl cellulose polymers present in the combination.

In a second embodiment of the above processes, the combination according to the invention is in the form of a gel. The combination is then mixed with the liquid organic compound and/or the liquid silicone to obtain a composition, at a temperature of at least 50°C until obtaining a homogeneous composition. The temperature is then cooled down to obtain a composition in a form of a gel.

Preferably, the temperature is lower than 90°C.

Preferably, the temperature is comprised between 50 and 90°C.

This embodiment is more adapted to thermal sensitive compounds, since the temperature doesn't need to exceed 90°C.

In the above processes, the weight ratio combination according to the invention / liquid organic compound is preferably of at least 10/90, more preferably of at least 20/80, even more preferably of at least 50/50.

In the above processes, the weight ratio combination according to the invention / liquid silicone is preferably of at least 50/50, more preferably of at least 60/40.

The present invention also relates to a composition in the form of a gel comprising a combination according to the invention, and a liquid organic compound, a liquid silicone and/or a solid compound.

The dimer diol, the branched primary alcohol of Formula (I), the glyceryl mono fatty acid ester and the ethyl cellulose polymer are as described above, including preferential and advantageous features.

The liquid organic compound is as described above, including preferential features.

In particular, the liquid organic compound is selected from the group consisting of propylene glycol, glycerol, 1,3 butylene glycol, polysorbate 20, polysorbate 80, polyglyceryl-3 diisostearate, mono and di-glyceride C18 unsaturated citrates, sorbitan mono oleate, triolein, isoamyl laurate, propylene glycol dicaprylate/dicaprate, medium chain triglycerides (MCT), paraffin highly liquid, octyldodecanol, caprylyl methicone, octocrylene, octyl methoxycinnamate, homosalate, ethylhexyl salicylate, ethyl butylacetylaminopropionate, citronellol, geraniol, citronella and mixtures thereof.

The solid compound may be a pigment, a UV filter, a gelling agent other than a combination according to the invention or a salt.

More particularly, the pigment is selected from the pigments usually used in cosmetics, such as titanium dioxide, sparkling powders, aluminum chlorohydrate and aluminum-zirconium.

More particularly, the UV filter is selected from butyl methoxydibenzoylmethane and benzophenone-3.

More particularly, the gelling agent other than a combination according to the invention is selected from the group consisting of dibutyl ethylhexanoyl glutamide and dibutyl lauroyl glutamide.

More particularly, the salt is selected from the group consisting of aluminium, zinc, sodium, magnesium, iodine, bromide, calcium and potassium salts.

In particular, the solid compound is selected from the group consisting of titanium dioxide, sparkling powders, butyl methoxydibenzoylmethane, benzophenone-3, dibutyl ethylhexanoyl glutamide, dibutyl lauroyl glutamide, and mixtures thereof.

Advantageously, in the composition according to the invention, the solid compound is a gelling agent other than a combination according the invention.

Preferably, the gelling agent other than a combination according to the invention is an alkyl glutamide.

The composition obtained is then in the form of a transparent stick, i.e. a transparent hard gel, as described in Example 2.3.

Preferably, the composition according to the invention comprises a mixture of alkyl glutamides, in particular dibutyl ethylhexanoyl glutamide and dibutyl lauroyl glutamide.

Preferably, the composition according to the invention comprises a liquid organic compound and a solid compound.

A particularly preferred composition according to the invention comprises a combination according to the invention, a liquid alcohol, preferably octyldodecanol, and alkyl glutamides, preferably dibutyl ethylhexanoyl glutamide and dibutyl lauroyl glutamide.

Preferably, the weight ratio combination according to the invention / alkyl glutamide(s) is comprised between 80/20 and 20/80, more preferably between 80/20 and 50/50.

The composition can be easily spread to the skin with a very good pay-off.

Indeed, its consistency is preferably comprised between 15 and 65, more preferably between 20 and 60 Nmm. Its hardness is preferably comprised between 10 and 65, more preferably between 12 and 60 N.

More particularly, the composition presents a consistency comprised between 50 and 65 Nmm and a hardness comprised between 12 and 20 N.

To obtain a composition according to the invention in the form of a gel, a combination according to the invention is mixed with a liquid organic compound, a liquid silicone and/or a solid compound.

In a first embodiment, the combination according to the invention is not in the form of a gel prior to the mixing with the liquid organic compound, a liquid silicone and/or a solid compound. It is thus necessary to heat the composition at a temperature higher than the glass transition temperature of the ethyl cellulose polymer present in the combination, or if more than one ethyl cellulose polymer is used, at a temperature higher than the highest glass transition temperature of the ethyl cellulose polymers present in the combination.

In a second embodiment, the combination according to the invention is in the form of a gel prior to the mixing with a liquid organic compound, a liquid silicone and/or a solid compound.

The invention also relates to a process for preparing a composition in the form of a gel comprising the following steps:
i) mixing a combination according to the invention (which is not in the form of a gel), and a liquid organic compound, a liquid silicone, a solid compound or a mixture thereof, to obtain a composition;
ii) heating the composition to a temperature higher than the glass transition temperature of the ethyl cellulose polymer present in the combination;
iii) cooling down the composition to obtain a composition in the form of a gel.

The dimer diol, the branched primary alcohol of Formula (I), the glyceryl mono fatty acid ester, the ethyl cellulose polymer, the liquid organic compound, the liquid silicone and the solid compound are as described above, including preferential and advantageous features.

In step ii), if more than one ethyl cellulose polymer is used, then the composition is heated at a temperature higher than the highest glass transition temperature of the ethyl cellulose polymers present in the combination.

The composition is preferably maintained at this temperature until complete solubilization of all ethyl cellulose polymer(s).

In a step iii), the temperature is cooled down preferably at a rate of 0.6°C/min or lower, until reaching a temperature of 45°C, preferably 40°C, more preferably 35°C.

The invention also relates to a process for preparing a composition in the form of a gel comprising the following steps:
i) bringing a combination in the form of a gel according to the invention to a temperature of at least 60°C;
ii) mixing the combination with a liquid organic compound, a liquid silicone, a solid compound or a mixture thereof;
iii) cooling down the composition to obtain a composition in the form of a gel.

The dimer diol, the branched primary alcohol of Formula (I), the glyceryl mono fatty acid ester and the ethyl cellulose polymer are as described above, including preferential and advantageous features.

Step ii) is preferably conducted at a temperature of at most 90°C, more preferably ot most 80°C. In particular, the temperature is comprised between 60 and 90°C. Step ii) is preferably conducted until a homogeneous composition is obtained.

The process for preparing a composition in the form of a gel can comprise the preparation of a combination in the form of a gel according to the process described above. The cooling of the combination of step iv) of the process for preparing a combination in the form of a gel, can then be stopped at a temperature of at least 60°C, preferably between 60 and 90°C, to start step ii) of the process for preparing a composition in the form of a gel.

The invention also concerns the use of a composition according to the invention, as a cosmetic product.

The cosmetic product can be in the form of a gel in different viscosity range.

The cosmetic product can be a waterless formulation.

The cosmetic product can be a make-up, a skin product, or a hair product, such as an eye shadow, a lipstick, a lip-gloss, an eye liner, a lip liner, a make-up remover, a face and body glitter gel, a face and/or body paint, a gelled baby oil, a massage oil, a deodorant, a sun protection product, an insect repellent product, a cleansing product, a cream, a diaper stick, a perfume stick, or a hair conditioner.

The invention is further described in the following examples, given by way of illustration, with reference to the figures:
Figure 1 represents a diagram relating to the evolution of the dynamic viscosity according to the increasing and decreasing temperature applied to the combination 1 at a shear rate of 10 s⁻¹;
Figure 2 represents a diagram relating to the evolution of the stress of combination 6 according to the shear rate applied.

### Materials used in Examples:

- liquid dimer diol: Radianol 1991 from Oleon (DD C36), which is a mixture of dimer diol isomers comprising 36 carbon atoms, obtained from dimerization of C18 fatty acids, wherein linear isomers content is of 76 wt% based on the weight of the mixture;
- liquid branched primary alcohols of Formula (I) (or Guerbet alcohols):
   ∘ 2-isopropyl-5-methylhexanol (isoC10): obtained from isoamyl alcohol;
   ∘ Isofol 18T from Sasol (C16-20), which is a mixture of 2-octyl-dodecanol (27-33wt%), 2-octyl-decanol (23-27wt%), 2-hexyl-dodecanol (23-27wt%) and 2-hexyl-decanol (15-20 wt%);
   ∘ isohexatriacontanol (isoC36): obtained from isostearyl alcohol;

### Preparation of Guerbet alcohols:

2-isopropyl-5-methylhexanol (isoC10) and isohexatriacontanol (isoC36) were respectively prepared from primary alcohols described above, according to a Guerbet reaction. Such a reaction is described in patent US 4,518,810 using KOH as a base and palladium as catalyst. The reaction medium were heated up to their respective boiling point.

Each crude reaction mixture was washed several times with demineralized water to remove all of the soaps. The washed products were subsequently filtered and dried under vacuum. Each remaining starting primary alcohol was separated by distillation. Then, each Guerbet alcohol was isolated by distillation.

The Guerbet alcohols thus obtained had each a purity of 98 wt%+/- 1.
- glyceryl mono fatty acid esters:
   ∘ glyceryl mono caprylate (GMC8), Jolee 7907 from Oleon;
   ∘ glyceryl mono caprate (GMC10) prepared according to the following protocol:

673 g of capric acid, 398 g of glycerol and 0.2 g of calcium hydroxide as catalyst were loaded into a 1L reactor equipped with a mechanical stirring paddle, a temperature sensor, a Vigreux column and a condenser. The water was removed from the reactor during the reaction. The reaction medium was mixed at 300 rpm and gradually heated up to 230°C under inert atmosphere (nitrogen) until an acid value of about 3 mg KOH/g was obtained, the acid value being measured according to standard AOCS Cd 3D-63. The temperature was then lowered to 210°C and 0.33 g of phosphoric acid was added to neutralize the catalyst. After 20 min the reaction medium was cooled to room temperature. Glycerol in excess and di- and tri-glycerides were removed by distillation to obtain monoglyceride of capric acid or glyceryl mono caprate with a purity of 72.7% by weight;
∘ mixture of glyceryl mono butyrate (C4), caprate (C8), caprylate (C10) and laurate (C12), (GMC4-12), Radiamuls 2101V from Oleon, which comprises 5-15 wt% of C4, 24-34 wt% of C8, 20-30 wt% of C10 and 30-40 wt% of C12;
∘ glyceryl mono oleate (GMC18:1) Radiamuls MG 2902K from Oleon;
   - Ethyl cellulose polymers:
      ∘ Ethocel Std. 20 Premium (EC 20 Prem);
      ∘ Ethocel Std. 45 Premium (EC 45 Prem);
      ∘ Ethocel Std.100 Premium (EC 100 Prem);
      ∘ Ethocel Std. 200 (EC 200);
where the number in the product name identifies the viscosity at 25°C of a solution of 5% by weight of Ethocel in a solvent, based on the weight of the solution, solvent being constituted by 80% toluene and 20% ethanol by weight; all of them being from Dow Chemical Company.

### Example 1: Preparation of combinations according to the invention in the form of a gel

Dimer diol, a branched primary alcohol of Formula (I) and a glyceryl mono fatty acid ester were mixed under mechanical stirring and heated up to 80°C. At this temperature the viscosity became low enough to create a vortex in the liquid.

An ethyl cellulose polymer was added bit by bit in the vortex.

Then the temperature was increased up to a temperature higher than the glass transition temperature of the ethyl cellulose polymer (the temperature was of 158°C when EC 100 Prem was used).

Once the combination became completely molten and transparent, it was cooled to room temperature.

A transparent gel was obtained.

The dimer diol, the branched primary alcohol of Formula (I), the glyceryl mono fatty acid ester and the ethyl cellulose polymer used in each combination are specified with their respective quantities in weight percentages, based on the weight of the composition, in Table 1 below.

**Table 1: Composition of the combinations according to the invention**

| | Dimer diol | Branched primary alcohol of Formula (I) | Glyceryl mono fatty acid ester | Ethyl cellulose polymer |
|---|---|---|---|---|
| Combination 1 | 32.8% DD C36 | 49.2% isoC36 | 10% GMC18:1 | 8% EC 100 Prem |
| Combination 2 | 32.8% DD C36 | 49.2% C16-20 | 10% GMC18:1 | 8% EC 100 Prem |
| Combination 3 | 32.8% DD C36 | 49.2% isoC10 | 10% GMC18:1 | 8% EC 100 Prem |
| Combination 4 | 32.8% DD C36 | 49.2% isoC36 | 10% GMC4-12 | 8% EC 100 Prem |
| Combination 5 | 32.8% DD C36 | 49.2% isoC36 | 10% GMC10 | 8% EC 100 Prem |
| Combination 6 | 32.8% DD C36 | 49.2% isoC36 | 10% GMC8 | 8% EC 100 Prem |
| Combination 7 | 32.8% DD C36 | 49.2% isoC36 | 10% GMC18:1 | 8% EC 20 Prem |
| Combination 8 | 32.8% DD C36 | 49.2% isoC36 | 10% GMC18:1 | 8% EC 45 Prem |
| Combination 9 | 32.8% DD C36 | 49.2% isoC36 | 10% GMC18:1 | 8% EC 200 |
| Combination 10 | 33.8% DD C36 | 50.2% isoC36 | 10% GMC18:1 | 6% EC 100 Prem |

### Example 2: Characteristics of combinations according to the invention in the form of a gel

As previously said, all combinations 1-12 according to the invention are transparent.

### 2.1 Dynamic viscosity at 25°C and at a shear rate of 10 s⁻¹

The dynamic viscosity of each combination was measured using a Discovery HR-1 Rheometer of TA instruments using a cone plate system, with a cone SST ST 40MM 2DEG Smart Swap, an angle of 2:00:03° and a truncation of 58 µm. For each combination, the dynamic viscosity was measured 24 hours after its preparation, at a shear rate of 10 s⁻¹ and at a temperature of 25°C.

Results are shown in Table 2 below.

**Table 2: Dynamic viscosity of combinations according to the invention**

| | Dynamic viscosity (mPa.s) at 25°C and at shear rate 10 s⁻¹ |
|---|---|
| Combination 1 | 146238 |
| Combination 2 | 98648 |
| Combination 3 | 98588 |
| Combination 4 | 129275 |
| Combination 5 | 93676 |
| Combination 6 | 81415 |
| Combination 7 | 47618 |
| Combination 8 | 76336 |
| Combination 9 | 105955 |
| Combination 10 | 55336 |

We can observe that the dynamic viscosity of the combination according to the invention is dependent on the quantity of each of its component.

Thus, the dynamic viscosity decreases, when the quantity of dimer diol decreases, when the quantity of ethyl cellulose polymer decreases (by comparing combinations 1 and 10), when the number of carbon atoms comprises in the fatty acid of the glycerol mono fatty acid ester(s) decreases (by comparing combinations 1, 5 and 6).

### 2.2 Dynamic viscosity - temperature relationship

Dynamic viscosities were measured using a Discovery HR-1 Rheometer of TA instruments using a cone plate system, with a cone SST ST 40MM 2DEG Smart Swap, an angle of 1:59:50° and a truncation of 53 µm.

For each combination, the dynamic viscosity was measured 24 hours after its preparation, at a shear rate of 10 s⁻¹ and at a temperature increasing by 1°C per minute, from 20° to 80°C. The measurement may have been stopped before reaching 80°C when dynamic viscosity became lower than 15000 mPa.s. Indeed, under 15000 mPa.s, the combination is no more a gel and the combination flows away as soon as the beaker is tilted.

The results are shown in Table 3 below.

**Table 3: Dynamic viscosity - temperature relationship of combinations 1-10 according to the invention**

| | Dynamic viscosity (mPa.s) at a shear rate of 10 s⁻¹ and at a temperature of | | | | | | |
|---|---|---|---|---|---|---|---|
| | 20°C | 30°C | 40°C | 50°C | 60°C | 70°C | 80°C |
| Combination 1 | 176457 | 93321 | 57544 | 49464 | 29627 | 14822 | 6779 |
| Combination 2 | 119632 | 79112 | 54120 | 29520 | 14523 | 7137 | 3373 |
| Combination 3 | 114360 | 65379 | 36178 | 16932 | 8118 | 4290 | 2333 |
| Combination 4 | 138092 | 57812 | 44660 | 29862 | 17760 | 10618 | 4919 |
| Combination 5 | 126083 | 81250 | 54411 | 40335 | 19511 | 5386 | 3017 |
| Combination 6 | 87384 | 45943 | 32296 | 27461 | 14380 | 6045 | 2830 |
| Combination 7 | 68159 | 33895 | 21189 | 10276 | 4484 | 2042 | 1092 |
| Combination 8 | 85425 | 60242 | 48551 | 30422 | 15707 | 7513 | 3187 |
| Combination 9 | 113764 | 40100 | 36605 | 26050 | 16126 | 10320 | 5580 |
| Combination 10 | 65894 | 39908 | 32700 | 21170 | 11681 | 1271 | 630 |

It can be observed than all combinations, except combination 7 that comprises a low viscous ethyl cellulose polymer, have a dynamic viscosity at a shear rate of 10 s⁻¹ greater than 15000 mPa.s at 50°C, meaning the combinations are still in the form of a gel at 50°C.

In addition, all combinations have a dynamic viscosity at a shear rate of 10 s⁻¹, lower than 15000 mPa.s at a temperature of 70°C, meaning the combinations are pourable at 70°C and they don't need to be heated at elevated temperatures to work with them in cosmetic products.

### 2.3 Stability

### 2.3.1 Over time

All combinations 1-10 are stable at room temperature, i.e. no sweating nor top separation are observable on the gel after at least 6 months.

Combinations 1 and 6 were submitted to a temperature of 60°C for 3 months, and no sweating nor top separation were observed once the combinations were cooled down to room temperature and recovered their initial viscosity.

Combinations according to the invention in the form of a gel are thus stable over time.

### 2.3.2 To temperature changes

For this test, dynamic viscosities were measured using a Discovery HR-1 Rheometer of TA instruments using a cone plate system, with a cone SST ST 40MM 2DEG Smart Swap, an angle of 1:59:50° and a truncation of 53 µm.

Combination 1 was subjected to a shear rate of 10 s⁻¹ and was scanned at increasing temperatures from 25 to 80°C and then at decreasing temperatures from 80 to 25°C. Combination 1 was thus subjected to three cycles of heating to 80°C followed by cooling to 25°C. Resulting dynamic viscosity curves are represented on Figure 1, with ramps 1 and 2, 3 and 4, 5 and 6 corresponding to the three cycles of heating (ramps 1, 3 and 5) and cooling (ramps 2, 4 and 6).

The combination according to the invention, has each time a dynamic viscosity lower than 10 Pa.s at 80°C, meaning it is pourable as such a temperature.

Moreover, the combination according to the invention recovered its initial viscosity at 25°C and its form of a gel, even after several repetitions of heating and cooling.

This demonstrates the stability of the gel towards repetitions of heating and cooling cycles and the capacity of the gel to rebuilt its structure after losing it at 80°C.

### 2.4 Shear-thinning property

Dynamic viscosities of combinations 1 and 6 were measured using a Discovery HR-1 Rheometer of TA instruments using a cone plate system, with a cone SST ST 40MM 2DEG Smart Swap, an angle of 1:59:50° and a truncation of 53 µm.

For each combination, the viscosity was measured 24 hours after its preparation, at 25°C, when the combination was subjected to a low shear rate going from 0.1 s⁻¹ to 300s⁻¹, over a period of 240 s, on the logarithmic scale (values close to zero corresponding to the Newtonian plateau, called "zero shear rate" viscosity), i.e. for which the viscosity is constant.

The results are shown in Table 4 below.

**Table 4: Dynamic viscosities at different shear rates of combinations 1 and 6 according to the invention**

| | | Dynamic viscosity (mPa.s) at shear rate: | | | |
|---|---|---|---|---|---|
| | zero shear rate | 10 s⁻¹ | 200 s⁻¹ | 250 s⁻¹ | 300 s⁻¹ |
| Combination 1 | 3402000 | 146238 | 11897 | 6580 | 5574 |
| Combination 6 | 2111000 | 87415 | 23125 | 13994 | 9698 |

It can be observed there that the dynamic viscosity of the combinations decreases, when the shear rate increases.

Combinations according to the invention are shear-thinning.

### 2.5 Thixotropic property

For this test, the same rheometer as previously described in Example 2.4 was used. Combination 6 according to the invention was subjected to a shear rate corresponding to an increase from 0.1 s⁻¹ to 300 s⁻¹, followed by a decrease from 300 s⁻¹ to 0.1 s⁻¹. The curves obtained, represented on Figure 2 shows the evolution of stress as a function of shear. It can be observed that the downward flow curve follows the same path than the upward flow curve. It means that the structure of the gel remains the same. The combination 6 is thixotropic.

### 2.6 Consistency and hardness

The consistency is a measure of the ease of penetration of external body, here a cylinder probe.

The hardness is a measure of the resistance of a material, here combination, to the penetration of an external body, here a cylinder probe.

The consistency and the hardness were measured with a Lloyd Instruments/Ametek TA1 Texture Analyzer and the Nexygen Plus V3 software, using the following characteristics: probe cylinder with diameter of 12 mm, area of probe of 113.1 mm², preload of 0.2 N (zero point), speed of going to zero point of 100 mm/min, speed during measurement of 20 mm/min, depth of 5 mm, force max of 50 N and temperature of 22°C.

Results are given in Table 5 below.

**Table 5: Consistency and hardness of combinations 1-10**

| | Consistency (Nmm) | Hardness (N) |
|---|---|---|
| Combination 1 | 39.00 | 16.70 |
| Combination 2 | 7.00 | 3.50 |
| Combination 3 | 1.95 | 0.60 |
| Combination 4 | 17.77 | 8.54 |
| Combination 5 | 41.40 | 16.20 |
| Combination 6 | 34.80 | 14.10 |
| Combination 7 | 3.71 | 1.32 |
| Combination 8 | 20.13 | 7.69 |
| Combination 9 | 29.80 | 15.00 |
| Combination 10 | 15.83 | 6.49 |

It can be observed by comparing combinations 1 and 10, that by lowering the quantity of ethyl cellulose polymer, the gel obtained is less viscous, and the values of consistency and of hardness decrease as well.

The consistency and the hardness decrease also with the viscosity of the ethyl cellulose from 100 to 20 mPa.s (combinations 1 and 7-8), and surprisingly with EC 200 the consistency and the hardness are lower than with EC 100 Prem (combinations 1 and 9).

The consistency and hardness are also dependent on the quantity of the primary branched alcohol of Formula (I): the more the quantity, the higher the consistency and the hardness.

The consistency and hardness are also dependent on the number of carbon atoms of the primary branched alcohol of Formula (I): the more the number of carbon atoms, the higher the consistency and the hardness (combinations 1-3).

The consistency and the hardness decrease with the increase in quantity of glyceryl mono fatty acid ester(s).

Depending on the application targeted, the man of the art can adapt the quantity of ethyl cellulose polymer, branched primary alcohol(s) of Formula (I), and glyceryl mono fatty acid ester in order to reach the viscosity, the consistency and the hardness desired.

### Example 3: Compositions according to the invention

### 3.1 With a liquid organic compound

### 3.1.1 Emollients

Different compositions were prepared by mixing combination 1 with:
- Esters
   ∘ glyceryl trioleate, Radia 7363 from Oleon;
   ∘ isoamyl laurate, Jolee 7750 from Oleon;
   ∘ propylene glycol dicaprylate/dicaprate, Radia 7208 from Oleon;
   ∘ caprylic/capric triglycerides, Radia 7104 from Oleon;
- a mineral oil: Paraffin highly liquid from Merck;
- a silicone oil: caprylyl methicone, Microcare Silicone M8100 from Thor;
with different weight ratios combination 1 according to the invention /emollient, at a temperature of 80°C until homogeneous.

Indeed, all liquid organic compounds tested when mixed to a combination according to the invention, led to homogeneous compositions.

Once cooled to room temperature, all compositions thus prepared increased significantly in viscosity while maintaining a transparent aspect.

Dynamic viscosities of compositions thus prepared were measured one day after their preparation following method described in Example 2.1.

Results are gathered in Tables 6 and 7 below.

**Table 6: Dynamic viscosities (mPa.s) at 25°C at 10 s⁻¹ shear rate of compositions**

| Emollient | Weight ratio combination 1 /emollient | | | | |
|---|---|---|---|---|---|
| | 0/100 | 20/80 | 40/60 | 60/40 | 80/20 |
| glyceryl trioleate | 40 | 343 | 2887 | 22226 | 73335 |
| isoamyl laurate | 4 | 39 | 1110 | 9780 | 38361 |
| propylene glycol dicaprylate/dicaprate | 5 | 105 | 1732 | 14140 | 86690 |
| caprylic/capric triglycerides | 5 | 192 | 2923 | 21590 | 70174 |

**Table 7: Dynamic viscosities (mPa.s) at 25°C, at 10 s⁻¹ shear rate of compositions**

| Emollient | Weight ratio combination 1 /emollient | | |
|---|---|---|---|
| | 0/100 | 60/40 | 80/20 |
| paraffin oil | 5 | 18740 | 59100 |
| silicone oil | 20 | 14567 | 59150 |

It can be observed that the combination according to the invention can thicken different emollients, such as esters, mineral oil and silicone oil, frequently used in cosmetic formulations. Depending on the ratio used, it is then possible to prepare compositions from low to high viscosities.

Two gel compositions were also prepared according to same method using 75 wt% of combination 6 and 25 wt% of silicone oil or paraffin oil. The gel compositions thus obtained are both shear-thinning, as illustrated by the decreasing viscosity values (Table 8 below) obtained by summiting the compositions to an increased shear stress.

**Table 8: Dynamic viscosities (mPa.s) at 25°C and at different shear rates**

| | Dynamic viscosity (mPa.s) at shear rate: | | | | |
|---|---|---|---|---|---|
| | zero shear rate | 10 s⁻¹ | 100 s⁻¹ | 200 s⁻¹ | 300 s⁻¹ |
| Combination 8 / Silicone oil = 75/25 | 107962 | 32301 | 6992 | 3983 | 3238 |
| Combination 8 / Paraffin oil = 75/25 | 62430 | 37550 | 11738 | 8242 | 6771 |

### 3.1.2 Humectants and emulsifiers

Compositions were prepared by mixing 10 wt% of :
- a polar humectant:
   ∘ propylene glycol, Radianol 4710 from Oleon;
   ∘ 1,3 butylene glycol from Daicel Corporation;
   or
- a polar emulsifier:
   ∘ polysorbate 20, Radiamuls SORB 2137K from Oleon;
   ∘ polysorbate 80, Radiamuls SORB 2157K from Oleon;
   ∘ polyglyceryl-3 diisostearate, Jolee 7245 from Oleon;
   ∘ mono and di-glyceride C18 unsaturated citrates, Radiamuls Citrem 2935K from Oleon;
   ∘ sorbitan mono oleate, Radia 2155 from Oleon;
with 90 wt% of combination 1 according to the invention at 80°C until homogeneous, weight percentages being based on the weight of the composition.

After cooling to room temperature, all compositions thus obtained were in the form of a transparent gel.

The combination according to the invention is then able to be also mixed homogeneously with polar compounds, such as with the alcohol and esters used in this Example and be used as gelling agent with such compounds.

Such transparent gels formed by mixing a combination and a polar emulsifier may be further emulsified with water such as during cleansing of skin.

After one day at room temperature, the dynamic viscosity, at 25°C and at a shear rate of 10 s⁻¹, of each composition was measured according to method described in Example 2.1. Results are gathered in Table 9 below.

**Table 9: Dynamic viscosities (mPa.s) at 25°C at a shear rate of 10 s⁻¹ of compositions**

| Humectant or emulsifier | Weight ratio 90/10 combination 3 / humectant or emulsifier |
|---|---|
| propylene glycol | 85550 |
| 1,3 butylene glycol | 94105 |
| polysorbate 20 | 82962 |
| polysorbate 80 | 89069 |
| polyglyceryl-3 diisostearate | 104293 |
| mono and di-glyceride C18 unsaturated citrates | 106035 |
| sorbitan mono oleate | 129755 |

### 3.1.3 Insect repellents

Compositions were prepared by mixing 80 wt% of combination 1 with 20 wt% of an insect repellent, respectively ethyl butylacetylaminopropionate (IR3535 from Merck) and citronella from VWR chemicals), at 80°C until homogeneous. After cooling down, gels were obtained. Their dynamic viscosity were measured according to method described in Example 2.1 and results are gathered in Table 10 below.

**Table 10: Dynamic Viscosity of repellent compositions**

| | Dynamic viscosity (mPa.s) at 25°C at 10 s⁻¹ shear rate |
|---|---|
| Combination 1 / IR3535 (80/20) | 44677 |
| Combination 1 / citronella (80/20) | 59885 |

Compositions obtained could be spread easily on the skin.
Thus, it is also possible to manufacture a gelled insect repellents.

### 3.1.4 Organic UV filters

Compositions were prepared by mixing, at 80°C until homogeneous, 90 wt% of combination 1 according to the invention with 10 wt% of an organic UV filter, respectively:
- octyl methoxycinnamate, Eusolex 2292 from Merck;
- octocrylene, Eusolex OCR from Merck;
- homosalate, Eusolex HMS from Merck;
- ethylhexyl salicylate, Eusolex OS from Merck.

All compositions thus prepared were transparent gels at 25°C.

It is thus possible to manufacture gelled sun protection compositions.

### 3.2 With a solid compound

### 3.2.1 Solid organic UV filter

A composition was prepared by mixing, at 80°C until homogeneous, 90 wt% of combination 1 according to the invention with 10 wt% of a solid organic UV filter, butyl methoxydibenzoylmethane, Eusolex 9020 from Merck.

After cooling to room temperature, un transparent gel was obtained.

This example shows that it is possible to form a transparent composition comprising a solid organic UV filter homogeneously dispersed in it.

### 3.2.2 Pigment

A composition was prepared by mixing at 80°C, 20 wt% of combination 1 with 30 wt% of propylene glycol dicaprylate/dicaprate (Radia 7208 from Oleon), and then adding 50 wt% of the pigment, titanium dioxide from Acros Organics. The composition was stirred at 80°C until homogeneous and was then allowed to cool down to room temperature.

A transparent gel was obtained, wherein the pigment was homogeneously dispersed.

The dynamic viscosity at 25°C and at a shear rate of 10 s⁻¹, measured as described in Example 2.1, was of 16150 mPa.s.

This example shows the good compatibility of the combination according to the invention with powder. It gives the possibilities to use the combination according to the invention in color cosmetic products.

### 3.3 With gelling agents

A composition was prepared by mixing, at 80°C until homogeneous, 75 wt% of combination 6 with 25 wt% of a gelling agent AJK-OD2046 from Ajinomoto composed of 8 wt% of dibutyl ethylhexanoyl glutamide, 12 wt% of dibutyl lauroyl glutamide and 80 wt% of octyldodecanol.

After cooling down, a transparent stick (consistent and hard composition) was obtained.

Its dynamic viscosity was measured according to method described in Example 2.1, and its consistency and hardness were measured according to method described in Example 2.6. Characteristics of the stick obtained are summarized in Table 11.

**Table 11: Characteristics of the stick**

| | Consistency (Nmm) | Hardness (N) | Dynamic viscosity (mPa.s) at shear rate 10 s⁻¹ | | |
|---|---|---|---|---|---|
| | | | 25°C | 40°C | 60°C |
| Stick | 57.9 | 16.7 | 117309 | 50063 | 3800 |

By comparing the consistency values of combination 6 and of the stick, it can be observed that those values increased significantly, by adding 25 wt% of gelling agent to 75 wt% of the combination 6.

This higher consistency (57.9 Nmm) means that the structure of the composition is more compact than the structure of the combination in the form of a gel.

The stick thus obtained presents an appearance of a solid, with a very good spreading and a very good pay off, when applied to the skin. The pay off is indeed easily controlled.

The dynamic viscosity slightly increased, compared to the dynamic viscosity of combination 8, and it is still around 50000 mPa.s at 40°C, meaning the stick does not melt at 40°C.

This stick could be used for the preparation of a stick in cosmetics to apply on the skin make-up, perfume, deodorant, skin/lips care, UV filters or insect repellent.

### Example 4: Cosmetic product according to the invention

### 4.1 Gelled baby oil

A gelled baby oil was prepared by mixing at 80°C until homogeneous, combination 1 and liquid organic compounds:
- Isoamyl laurate as described previously;
- Glycerine: Glycerine 4810 from Oleon;
- Isopropyl palmitate: Radia 7732 from Oleon;
- Antimicrobial agent: Phenonip from Clariant;
- Perfume: Avallon code zz13419 from SGP Selin.

Quantities of each compounds are described in Table 12 below.

Once cool down to room temperature a transparent gel was obtained, with a good skin feel.

**Table 12: Gelled baby oil**

| | Function | Gelled baby oil (%w/w) |
|---|---|---|
| Combination 1 | gelling agent | 70 |
| Isoamyl laurate | emollient | 24.7 |
| Glycerine | humectant | 3 |
| Isopropyl palmitate | emollient | 2 |
| Phenonip | antimicrobial agent | 0.1 |
| Perfume | perfume | 0.2 |

The dynamic viscosity at 25°C and at a shear rate of 10 s⁻¹, measured as described in Example 2.1, was of 23483 mPa.s.

### 4.2 Make-up remover

A make-up remover was prepared by mixing at 80°C until homogeneous, combination 1 and liquid organic compounds:
- triheptanoin: Radia 2375 from Oleon;
- propylene glycol dicaprylate/dicaprate, Radia 7208 from Oleon;
- isoamyl laurate, Jolee 7750 from Oleon.

Quantities of each compounds are described in Table 13 below.

**Table 13: Make-up remover**

| | Function | Make-up remover (%w/w) |
|---|---|---|
| Combination 1 | thickener | 50 |
| Triheptanoin | emollient | 10 |
| Propylene glycol dicaprylate/dicaprate | emollient | 10 |
| Isoamyl laurate | emollient | 30 |

The dynamic viscosity at 25°C and at a shear rate of 10 s⁻¹, measured as described in Example 2.1, was of 6453 mPa.s.

The make-up remover obtained exhibit a good skin feel with a good spreading.

### 4.3 Sun protection product

By mixing some of the previous liquid and solid organic UV filters with a combination according to the invention (used as thickener) and an emollient, transparent compositions with a wide range in SPF and UV spectrum as illustrated by sun protection compositions whose contents are described in Table 14 below, can also be obtained.

**Table 14: Sun protection compositions**

| | SPF 25 (%w/w) | SPF 50 (%w/w) |
|---|---|---|
| Combination 1 | 55 | 50 |
| Propylene glycol dicaprylate/dicaprate | 25 | 10 |
| Octyl methoxycinnamate | 5 | 10 |
| Butyl methoxydibenzoylmethane | 5 | 5 |
| Benzophenone-3 | 5 | |
| Ethylhexyl salicylate | 5 | 5 |
| Homosalate | | 10 |
| Octocrylene | | 10 |

The dynamic viscosities at 25°C and at a shear rate of 10 s⁻¹, measured as described in Example 2.1, were of 8502 mPa.s for SPF 25 and of 12346 mPa.s for SPF 50.

Both sun protection compositions can be easily spread on the skin.

### 4.4 Face and body glitter gel

A face and body gel was prepared by mixing at 80°C until homogeneous, combination 1 with propylene glycol dicaprylate/dicaprate (Radia 7208 from Oleon) and a golden-brown sparkling powder, Colorona glitter bronze from Merck, according to quantities described in Table 15.

**Table 15: Face and body glitter gel**

| | Function | Face and body glitter gel (%w/w) |
|---|---|---|
| Combination 1 | gelling agent | 80 |
| Propylene glycol dicaprylate/dicaprate | emollient | 19 |
| Colorona glitter bronze | golden-brown sparkling powder | 1 |

At room temperature, the cosmetic product was in the form of a gel with a well dispersed sparkling powder. This gave a shiny product with a nice uniform color reflection. and a good spreading on the skin.

The dynamic viscosities at 25°C and at a shear rate of 10 s⁻¹, measured as described in Example 2.1, were of 59594 mPa.s. The cosmetic product exhibits a good spreading on the skin.

## Claims

1. Combination comprising or consisting of:
- a dimer diol;
- branched primary alcohol(s) of Formula (I), 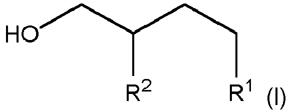 wherein R¹ and R², identical or different, are linear or branched alkyl groups, comprising from 3 to 16 carbon atoms;
- at least 5% by weight of a glyceryl mono fatty acid ester; and
- at least 3% by weight of an ethyl cellulose polymer;
wherein the dimer diol and the branched primary alcohol(s) of Formula (I) are liquid at 25°C and atmospheric pressure; and
wherein the total quantity of dimer diol and branched primary alcohol of Formula (I) is of at least 70% by weight;
weight percentages being based on the weight of the combination.

2. Combination according to claim 1, wherein the weight ratio total quantity of branched primary alcohol(s) of Formula (I) / quantity of dimer diol is comprised between 0.5 and 2.2.

3. Combination according to claim 1 or 2, wherein the combination is in the form of a gel.

4. Process for preparing a combination in the form of a gel according to claim 3, comprising the following steps:
i) mixing a dimer diol, branched primary alcohol(s) and glyceryl mono fatty acid ester(s) at a temperature of at least 40°C;
ii) adding an ethyl cellulose polymer to obtain a combination;
iii) heating the combination under stirring at a temperature higher than the glass transition temperature of the ethyl cellulose polymer; and
iv) cooling down the combination to obtain a combination in the form of a gel.

5. Use of a combination according to any of claims 1 to 3, as a thickener.

6. Process for increasing the viscosity of a liquid organic compound and/or a liquid silicone, by mixing a combination according to any of claims 1 to 3, with said liquid organic compound and/or liquid silicone.

7. Use of a combination according to any of claims 1 to 3, as a shear-thinning agent.

8. Process for decreasing the viscosity under shear-stress of a liquid organic compound and/or a liquid silicone, by mixing a combination according to any of claims 1 to 3 with said liquid organic compound and/or liquid silicone.

9. Use of a combination according to any of claims 1 to 3, as a gelling agent.

10. Process for gelling a liquid organic compound and/or a liquid silicone, comprising a step of mixing and heating a combination according to any of claims 1 to 3, with said liquid organic compound and/or liquid silicone.

11. Composition in the form of a gel comprising a combination according to any of claims 1 to 3, and a liquid organic compound, a liquid silicone and/or a solid compound.

12. Composition according to claim 11, wherein the solid compound is a gelling agent other than a combination according to any of the claims 1 to 3.

13. Process for preparing a composition in the form of a gel comprising the following steps:
i) mixing a combination according to claim 1 or 2, and a liquid organic compound, a liquid silicone, a solid compound or a mixture thereof, to obtain a composition;
ii) heating the composition to a temperature higher than the glass transition temperature of the ethyl cellulose polymer present in the combination;
iii) cooling down the composition to obtain a composition in the form of a gel.

14. Process for preparing a composition in the form of a gel comprising the following steps:
i) bringing a combination in the form of a gel according to claim 3, to a temperature of at least 60°C;
ii) mixing the combination with a liquid organic compound, a liquid silicone, a solid compound or a mixture thereof;
iii) cooling down the composition to obtain a composition in the form of a gel.

15. Use of a composition according to claim 11 or 12, as a cosmetic product.
